# FASCICULE DE BREVET EUROPEEN

(11) **EP 0 748 785 B1**
(45) Date de publication et mention de la délivrance du brevet: **31.05.2000**
(21) Numéro de dépôt: 96401185.2
(22) Date de dépôt: 04.06.1996
(51) Int. Cl.: C07C 5/25

(54) **Procédé pour le déplacement de la double liaison des oléfines à l'aide d'une composition catalytique à base de complexes de métaux de transition**
Verfahren zur Verlegung der Doppelbindung in Olefinen in Gegenwart von einer, ein Übergangsmetallcomplex enthaltenden, Katalysatorzusammensetzung
Process for moving the double bound in olefins using a catalytic composition comprising transition metal complexes

(30) Priorité: 16.06.1995 FR 9507329
(43) Date de publication de la demande: 18.12.1996
(73) Titulaire: INSTITUT FRANCAIS DU PETROLE, 92502 Rueil-Malmaison (FR)
(72) Inventeur: Chauvin, Yves, 78230 Le Pecq (FR); Mussmann, Lothar, 63457 Hanau-Wolfgang (DE); Olivier, Hélène, 92500 Rueil Malmaison (FR)

(56) Documents cités:
- US-A- 3 565 823
- US-A- 3 657 368
- US-A- 3 832 391

## Description

L'objet de la présente invention est un procédé pour l'isomérisation, c'est-à-dire le déplacement de la double liaison, des oléfines à l'aide d'une composition catalytique résultant de l'interaction d'un sel organique-inorganique liquide à la température de réaction, qu'on appellera par la suite "sel fondu", et d'un complexe d'un métal de transition des groupes 8, 9 et 10.

On connait un très grand nombre de complexes de métaux de transition des groupes 8, 9 et 10 susceptibles de catalyser en catalyse homogène le déplacement de la double liaison des oléfines et qui sont solubles dans des solvants organiques compatibles, ou dans les réactifs et dans les produits issus de l'isomérisation. Ces catalyseurs ont fait l'objet d'un article dans Principle and Applications of Organotransition Metal Chemistry de J.P. Collman, University Science Books, Mill Valley, U.S.A.

Il a été décrit dans le brevet U.S. 3.565.823 une composition consistant en une dispersion constituée d'un composé notamment d'un métal de transition dans un sel d'étain ou de germanium et d'un ammonium ou d'un phosphonium quaternaire. Dans les brevets U.S. 3.657.368 et U.S. 3.832.391 ont été décrit des procédés d'hydrogénation, d'isomérisation et de carbonylation des oléfines et dans le brevet U.S. 3.919.271 un procédé d'hydrogénation des nitriles utilisant tous deux la composition précédente, procédé qui est également décrit dans les brevets U.S. 3.832.391 et US.3.657.368.

Les compositions précédemment décrites présentent le désavantage d'avoir un point de fusion relativement élevé.

Il a maintenant été trouvé que les complexes de métaux de transition des groupes 8, 9 et 10 en particulier les complexes du ruthénium, du rhodium, de l'iridium, du palladium et du platine, associés à un sel organique-inorganique liquide à basse température sont susceptibles d'isomériser les oléfines.

La composition catalytique selon l'invention comprend au moins un composé d'au moins un métal de transition des groupes 8, 9 et 10 en particulier les complexes du ruthénium, du rhodium, de l'iridium, et aussi du nickel, du palladium et du platine et au moins un sel d'ammonium et/ou de phosphonium quaternaire, dit "sel fondu" ladite composition résultant de la dissolution, du moins en partie, d'un composé d'un métal de transition dans un "sel fondu" .

L'objet de l'invention est un procédé pour l'isomérisation des oléfines, procédé dans lequel la ou les oléfines sont mises au contact d'au moins un composé d'au moins un métal de transition des groupes 8, 9 et 10 en particulier du ruthénium, du rhodium, de l'iridium, et aussi du palladium, du platine et du nickel, ledit composé étant dissous au moins en partie dans un "sel fondu". Ledit milieu "sel fondu" est à base d'un cation organique et d'un anion minéral. Les produits issus de l'isomérisation sont peu ou pas solubles dans la composition catalytique.

Les "sels fondus" selon l'invention ont pour formule générale Q⁺ A⁻ dans laquelle Q⁺ représente un ammonium quaternaire et/ou d'un phosphonium quaternaire et A- représente tout anion connu susceptible de former un sel liquide à basse température c'est-à-dire en dessous de 150°C et avantageusement d'au plus 80° C, et de préférence en dessous 50°C. Ces anions sont choisis dans le groupe formé par les ions tétrafluoroborate, tétrachloroborate, hexafluorophosphate, hexafluoroantimonate, hexafluoroarsénate, trifluorométhylsulfonate, fluorosulfonate, tétrachloroaluminate, dichlorocuprate, trichlorozincate. Aux anions A⁻ et en particulier à ceux cités ci-dessus il est possible d'ajouter des anions choisis dans le groupe formé par le trichlorocuprate, le tétrachlorocuprate, l'heptachloroaluminate, le décachloroaluminate. Les ammonium et/ou phosphonium quaternaires répondent de préférence aux formules générales NR¹R²R³R⁴⁺ et PR¹R²R³R⁴⁺, ou aux formules générales R¹R²N=CR³R⁴⁺ et R¹R²P=CR³R⁴⁺ où R¹, R², R³ et R⁴, identiques ou différents, représentent l'hydrogène à l'exception du cation NH₄⁺ et de préférence un seul substituant peut représenter l'hydrogène, ou des restes hydrocarbyles ayant de 1 à 12 atomes de carbone, par exemple des groupements alkyles, saturés ou non saturés, cycloalkyls ou aromatiques, aryl ou aralkyl, comprenant de 1 à 12 atomes de carbone. Les ammonium et/ou phosphonium peuvent également être dérivés d'hétérocycles azotés ou phosphorés comportant 1, 2 ou 3 atomes d'azote et/ou de phosphore, de formule générales: 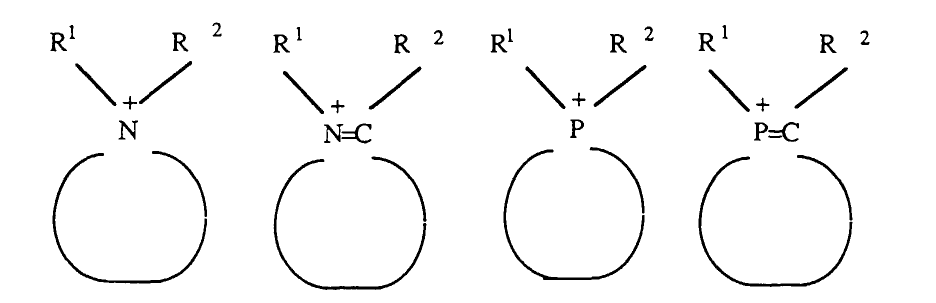 dans lesquelles les cycles sont consitués de 4 à 10 atomes, de préférence 5 à 6 atomes, R¹ et R² étant définis comme précédemment. L'ammonium ou le phosphonium quaternaire peut également être un cation de formule:

R¹R²⁺N=CR³-R⁵-R³C=N⁺R¹R²

R¹R²⁺P=CR³-R⁵-R³C=P⁺R¹R²

dans laquelle R¹,R²,R³, identiques ou différents sont définis comme précédemment et R⁵ représente un reste alkylène ou phénylène. Parmi les groupements R¹, R², R³ et R⁴ on mentionnera les radicaux méthyl, éthyl, propyl, isopropyl, butyl, secondaire butyl, tertiaire butyl, amyl, méthylène, éthylidène, phényl ou benzyl; R⁵ pourra être un groupement méthylène, éthylène, propylène ou phénylène. Le cation ammonium et/ou phosphonium est choisi de préférence dans le groupe formé par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, l'éthyl-3 méthyl-1 imidazolium, le tétrabutylphosphonium. A titre d'exemples des sels utilisables selon l'invention on peut citer l'hexafluorophosphate de N-butylpyridinium, le tétrafluoroborate de N-éthyl pyridinium, le tétrafluoroborate de tétrabutylphosphonium, l'hexafluoroantimonate de butyl-3 méthyl-1 imidazolium, l'hexafluorophosphate de butyl-3 méthyl-1 imidazolium, le trifluorométhylsulfonate de butyl-3 méthyl-1 imidazolium , le fluorosulfonate de pyridinium, l'hexafluorophosphate de triméthylphénylammonium, le tétrachloroaluminate de butyl-3 méthyl-1 imidazolium, l'heptachloroaluminate de butyl-3 méthyl-1 imidazolium, le chlorure de triméthylphényl ammonium, le chlorure d'éthyl-3 méthyl-1 imidazolium, le bromure de tétrabutylphosphonium, le chlorure de N-butylpyridinium, le bromure de N-éthylpyridinium, le chlorure de butyl-3 méthyl-1 imidazolium, le chlorure de diéthylpyrazolium, le chlorhydrate de pyridinium. Ces sels peuvent être utilisés seuls ou en mélange. Ils ont une fonction de solvant.

Les composés des métaux de transition utilisables selon l'invention sont de façon générale tous les complexes des métaux de transition connus de l'homme de l'art. Ce sont des composés zéro, mono, di ou trivalents dans lesquels le métal est choisi dans le groupe formé par Fe, Ru, Os, Co, Rh, Ir, Ni, Pd, Pt et de préférence Ru, Rh et Ir, peut être lié par exemple à des ions halogénures, hexafluorophosphosphate, hexafluoroarsenate, tétrafluoroborate, tétrachloborate etc..., hydrure, et des ligands ioniques par exemple hydrocarbonés tels que cyclopentadiényles et cyclopentadiényles substitués, acétylacétonates et acétylacétonates substitués, ou encore à des ligands neutres tels les phosphines tertiaires, les diphosphines ditertiaires, les phosphites, les oléfines, l'oxyde de carbone, les nitriles. Ces complexes selon l'invention peuvent être mononucléaires ou polynucléaires, neutres ou ioniques. Ils peuvent renfermer un ligand chiral. A titre d'exemple parmi les complexes utilisables selon l'invention on peut citer RuH₄(PPh₃)₂, RhCl(PPh₃)₃, [Rh(norbornadiène)(PPh₃)₂]⁺ [PF₆]⁻, [Rh(norbornadiène)(PPh₃)₂]⁺ [BF₄]⁻, [Rh(norbornadiène)(PPh₃)₂]⁺ [ClO₄]⁻, lrCl(PPh₃)₃, HRh(CO)(PPh₃)₃, (C₅H₅)RhCl₂, Rh(C₅Me₅)Cl₂(Ph₂PCH₂COPh), IrCl(CO)(PPh₃)₂, [Rh(norbornadiène)(PPh₃)(Ph₂PCH₂COPh)]⁺[PF₆]⁻, [Rh(norbornadiène)(bisdiphénylphosphinoéthane)]⁺[PF₆]⁻, [Rh(norbornadiène)(bisdiphénylarsinoéthane)]⁺[PF₆]⁻, [Rh(norbornadiène)(DIOP)]⁺[PF₆]⁻,[Ru(norbornadiène)(C₅Me₅)]⁺[BF₄]⁻, [(C₆Me₆)RuCl₂]₂, [(C₆H₆)RuCl₃Ru(C₆H₆)]PF₆, [RhCl(cyclooctène)₂].

Ces complexes peuvent être préparés en dehors du milieu réactionnel et y être introduits pour la réaction. Ils peuvent également être formés in situ, dans le milieu réactionnel, par introduction des composants nécessaires à leur formation. D'autres composés des métaux de transition sont utilisables, tels que les sels inorganiques (halogénures, par exemple : chlorure, bromure, iodure) oxydes, hydroxydes, des sels organiques.

D'une façon générale, la composition catalytique peut contenir un solvant organique comme les hydrocarbures aromatiques ou des composés hydrocarbonés. De façon préférée, la composition catalytique ne contient pas d'eau.

La concentration du composé (du complexe de préférence) de métal de transition dans le "sel fondu" est avantageusement comprise entre 1 mmole de composé par litre de "sel fondu" et 500 mmoles par litre, de préférence entre 2 et 200 mmoles par litre, et encore entre 2 et 100, voire 2 à 50.

Les composés entrant dans la composition selon l'invention, peuvent être mélangés dans un ordre quelconque, à une température comprise entre -20°C et + 200°C, et de préférence -20°C à 140°C et avantageusement de 0°C à 120°C, voire 0 à 80°C ou 0 à moins de 50°C.

Les oléfines susceptibles d'être isomérisées sont par exemple 1-butène, le 2-butène, le 1-pentène, 2-pentène, le 2-méthyl-2-butène, le 2-méthyl-1-butène, les 4-méthyl-2-pentènes, le 4-méthyl-1-pentène, le 2-méthyl-1-pentène, le 1-hexène, les 2-hexènes, les 3-hexènes. Plus généralement, les oléfines peuvent être linéaires, ramifiées, cycliques.

Dans le procédé d'isomérisation, l'oléfine pourra être utilisée pure ou diluée par des hydrocarbures saturés ou insaturés tels que ceux qu'on trouve dans les "coupes" issues des divers procédés de raffinage des hydrocarbures comme par exemple les butanes avec les butènes.

La température à laquelle se fera l'isomérisation sera comprise entre -10°C et 200°C, avantageusement la température est inférieure à 150°C, de préférence entre +10°C et moins de 150°C. La pression totale ou partielle d'hydrogène peut être comprise entre la pression atmosphérique ou une pression inférieure à la pression atmosphérique, et 20 MPa, de préférence entre la pression atmosphérique et 10 MPa.

Avant de mettre la charge d'oléfine(s) au contact de la composition catalytique, ladite composition est si besoin (non nécessaire dans le cas des hydrures par exemple) activée par de l'hydrogène à une température comprise entre -10°C et 200°C, avantageusement la température est inférieure à 150°C, de préférence entre +10°C et moins de 150°C.

Avantageusement la réaction d'isomérisation a lieu sous atmosphère inerte ne contenant que peu, le rapport molaire hydrogène/hydrocarbure étant inférieur à 0.01 ou pas d'hydrogène.

La réaction catalytique d'hisomérisation des oléfines peut être conduite en système fermé, en système semi-ouvert ou en continu avec un ou plusieurs étages de réaction. A la sortie du réacteur la phase organique contenant les produits de réaction est séparée avantageusement par simple décantation de la phase polaire catalytique contenant le "sel fondu" et la majeure partie du catalyseur. La phase polaire qui contient au moins en partie le catalyseur, est, au moins en partie, retournée au réacteur d'isomérisation, l'autre partie étant traitée pour éliminer les résidus du catalyseur.

L' exemple suivant illustre l'invention sans en limiter la portée:

### EXEMPLE

Dans un réacteur en verre à pression d'une contenance de 50 mL purgé de l'air et de l'humidité et placé sous la pression atmosphérique d'hydrogène, on a introduit 4mL de dichlorocuprate de butylméthylimidazolium, 43,2 mg (0.05 mmole) du complexe [Rh(norbornadiène)(PPh₃)₂]⁺PF₆⁻. On a porté la pression d'hydrogène à 0,1 MPa et la température à 30°C pendant 10 minutes. Le sel devient orangé. On retourne à pression atmosphérique et on injecte 2 mL de 1-pentène et on met le système d'agitation en route sous atmosphère d'hydrogène. Après 2 heures d'agitation on a laissé se décanter le mélange; on a soutiré la phase hydrocarbonée surnageante. La conversion du 1-pentène en 2-pentène était de 19 %, sans formation de pentane. On a recommencé la même réaction avec le sel ayant précédemment servi, balayé à l'hydrogène puis mis sous atmosphère d'argon. Une nouvelle charge de 1-pentène a été convertie en 91% de 2-pentène, à 25°C pendant 17 heures, sans formation de pentane.

## Revendications

1. Procédé pour l'isomérisation de la double liaison des oléfines dans lequel la dite oléfine est mise au contact d'une composition catalytique comprenant au moins un composé d'au moins un métal de transition des groupes 8, 9 et 10 et au moins un sel d'ammonium et/ou de phosphonium quaternaire de formule générale Q⁺A⁻, dans laquelle Q⁺ représente un ammonium quaternaire et/ou phosphonium quaternaire, et A⁻ représente un anion choisi dans le groupe formé par le tétrafluoroborate, le tétrachloroborate, l'hexafluorophosphate, l'hexafluoroantimonate, l'hexafluoroarsénate, le dichloro cuprate, le tétrachloroaluminate, le trifluorométhylsulfonate, le fluorosulfonate, le trichlorozincate.

2. Procédé selon l'une des revendications précédentes dans lequel la composition catalytique comprend en outre au moins un anion choisi dans le groupe formé par l'heptachloroaluminate, le décachloroaluminate, le trichlorocuprate, le tétrachlorocuprate.

3. Procédé selon l'une des revendications précédentes dans lequel le cation ammonium et/ou phosphonium quaternaires sont choisis dans le groupe formé par les cations de formule générale:
R¹R²R³R⁴N⁺
R¹R²N=CR³R⁴⁺
R¹R²R³R⁴P⁺
R¹R²P=CR³R⁴⁺ 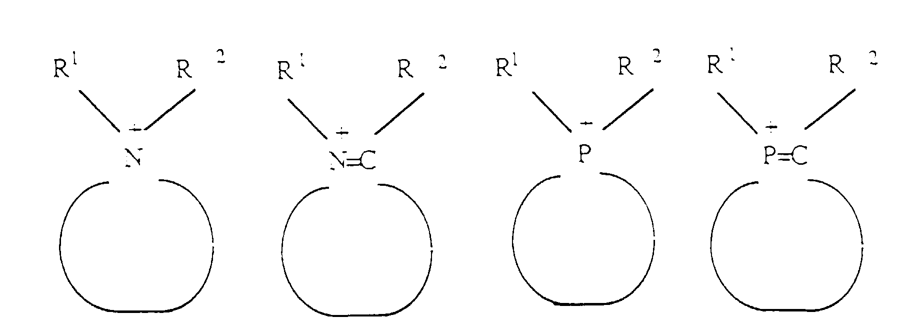
dans lesquelles R¹,R²,R³,R⁴, identiques ou différents représentent l'hydrogène, à l'exception de NH₄⁺, et des restes hydrocarbyles ayant 1 à 12 atomes de carbone, et dans lesquelles les cycles sont constitués de 4 à 10 atomes.

4. Procédé selon l'une des revendications précédentes dans lequel le cation ammonium et/ou phosphonium quaternaires ont pour formules générales:
R¹R²⁺N=CR³-R⁵-R³C=N⁺R¹R²
R¹R²⁺P=CR³-R⁵-R³C=P⁺R¹R²
dans laquelle R^{1,}R²,R³ identiques ou différents représentent l'hydrogène ou des restes hydrocarbyles ayant 1 à 12 atomes de carbone, et R⁵ représente un reste alkylène ou phénylène.

5. Procédé selon l'une des revendications précédentes dans lequel le cation ammonium et/ou phosphonium quaternaires sont choisis dans le groupe constitué par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, l'éthyl-3 méthyl-1 imidazolium, le tétrabutylphosphonium.

6. Procédé selon l'une des revendications précédentes dans lequel les sels d'ammonium et/ou de phosphonium quaternaires sont choisis dans le groupe constitué par l'hexafluorophosphate de N-butylpyridinium, le tétrafluoroborate de N-éthyl pyridinium, le tétrafluoroborate de tétrabutylphosphonium, l'hexafluoroantimonate de butyl-3 méthyl-1 imidazolium, l'hexafluorophosphate de butyl-3 méthyl-1 imidazolium, le trifluorométhylsulfonate de butyl-3 méthyl-1 imidazolium , le fluorosulfonate de pyridinium, l'hexafluorophosphate de triméthylphénylammonium, le tétrachloroaluminate de butyl-3 méthyl-1 imidazolium, l'heptachloroaluminate de butyl-3 méthyl-1 imidazolium, le chlorure de triméthylphényl ammonium, le chlorure d'éthyl-3 méthyl-1 imidazolium, le bromure de tétrabutylphosphonium, le chlorure de N-butylpyridinium, le bromure de N-éthylpyridinium, le chlorure de butyl-3 méthyl-1 imidazolium, le chlorure de diéthylpyrazolium, le chlorhydrate de pyridinium.

7. Procédé selon l'une des revendications précédentes dans lequel le métal de transition est choisi dans le groupe formé par le ruthénium, le rhodium, l'iridium.

8. Procédé selon l'une des revendications précédentes dans lequel le composé de métal de transition est un complexe de métal de transition.

9. Procédé selon l'une des revendications précédentes caractérisée en ce que le métal est lié à des ligands neutres ou ioniques.

10. Procédé selon l'une des revendications précédentes dans lequel les complexes des métaux des groupes 8 et 9 sont choisis dans le groupe formé par RuH₄(PPh₃)₂, RhCl(PPh₃)₃, [Rh(norbornadiène)(PPh₃)₂]⁺ [PF₆]⁻, IrCl(PPh₃)₃, HRh(CO)(PPh₃)₃, (C₅H₅)RhCl₂, IrCl(CO)((PPh₃)₂, [Rh(norbornadiène)(PPh₃)(Ph₂PCH₂COPh)]⁺[PF₆]⁻ [Rh(norbornadiène)(bisdiphénylphosphinoéthane)]⁺[PF₆]⁻, [Rh(norbornadiène)(bisdiphénylarsinoéthane)]⁺[PF₆]⁻, [Ru(norbornadiène)(C₅Me₅)]+[BF₄]⁻, [(C₆Me₆)RuCl₂]₂, [(C₆H₆)RuCl₃Ru (C₆H₆)]⁺PF₆-,

11. Procédé selon l'une des revendications précédentes dans lequel la concentration du composé des métaux de transition des groupes 8 à 10 par rapport au sel d'ammonium et/ou de phosphonium est de 1 à 500 mmoles par litre.

12. Procédé selon l'une des revendications précédentes dans lequel la composition catalytique comprend un solvant organique.

13. Procédé selon l'une des revendications précédentes dans lequel les oléfines sont le 1-butène, le 2-butène, le 1-pentène, 2-pentène, le 2-méthyl-2-butène, le 2-méthyl-1-butène, les 4-méthyl-2-pentènes, le 4-méthyl-1-pentène, le 2-méthyl-1-pentène, le 1-hexène, les 2-hexènes, les 3-hexènes.

14. Procédé selon l'une des revendications précédentes dans lequel la phase hydrocarbonée est séparée de la phase polaire, ladite phase polaire contenant au moins une partie du catalyseur étant au moins en partie recyclée dans le réacteur d'isomérisation.

15. Procédé selon l'une des revendications précédentes opérant entre -10 et 200°C, sous une pression comprise entre 0.1 MPa et 20 MPa.

## Patentansprüche

1. Verfahren zur Isomerisierung der Doppelbindung von Olefinen, bei dem man das genannte Olefin mit einer katalytischen Zusammensetzung in Kontakt bringt, die mindestens eine Verbindung mindestens eines Übergangsmetalls der Gruppen 8, 9 und 10 des PSE und mindestens ein quaternäres Ammonium- und/oder Phosphoniumsalz der allgemeinen Formel Q⁺A⁻ enthält, worin Q⁺ für quaternäres Ammonium und/oder quaternäres Phosphonium und A⁻ für ein Anion, ausgewählt aus der Gruppe, die besteht aus Tetrafluoroborat, Tetrachloroborat, Hexafluorophosphat, Hexafluoroantimonat, Hexafluoroarsenat, Dichlorocuprat, Tetrachloroaluminat, Trifluoromethylsulfonat, Fluorosulfonat und Trichlorozinkat, stehen.

2. Verfahren nach einem der vorhergehenden Ansprüche, in dem die katalytische Zusammensetzung außerdem mindestens ein Anion, ausgewählt aus der Gruppe, die besteht Heptachloroaluminat, Decachloroaluminat, Trichlorocuprat und Tetrachlorocuprat, enthält.

3. Verfahren nach einem der vorhergehenden Ansprüche, in dem das quaternäre Ammonium- und/oder Phosphoniumkation ausgewählt wird aus der Gruppe, die besteht aus Kationen der allgemeinen Formel:
R¹R²R³R⁴N⁺
R¹R²N=CR³R⁴⁺
R¹R²R³R⁴P⁺
R¹R²P=CR³R⁴⁺ 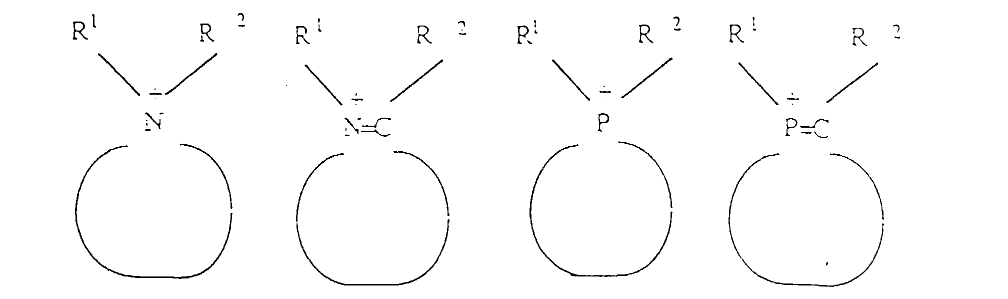
worin bedeuten R¹, R², R³ und R⁴, die gleich oder verschieden sind, Wasserstoff, mit Ausnahme von NH₄⁺, und Hydrocarbylreste mit 1 bis 12 Kohlenstoffatomen, und in denen die Cyclen aus 4 bis 10 Atomen bestehen.

4. Verfahren nach einem der vorhergehenden Ansprüche, in dem das quaternäre Ammonium- und/oder Phosphoniumkation die allgemeinen Formeln haben:
R¹R²⁺N=CR³-R⁵-R³C=N⁺R¹R²
R¹R²⁺P=CR³-R⁵-R³C=P+R¹R²
worin bedeuten R¹, R² und R³, die gleich oder verschieden sind, Wasserstoff oder Hydrocarbyl-Reste mit 1 bis 12 Kohlenstoffatomen und R⁵ einen Alkylenoder Phenylen-Rest.

5. Verfahren nach einem der vorhergehenden Ansprüche, in dem das quaternäre Ammonium- und/oder Phosphoniumkation ausgewählt werden aus der Gruppe, die besteht aus N-Butylpyridinium, N-Ethylpyridinium, 3-Butyl-1-methyl-imidazolium, Diethylpyrazolium, 3-Ethyl-1-methyl-imidazolium, Pyridinium, Trimethylphenylammonium, 3-Ethyl-1-methyl-imidazolium und Tetrabutylphosphonium.

6. Verfahren nach einem der vorhergehenden Ansprüche, in dem die quaternären Ammonium- und/oder Phosphoniumsalze ausgewählt werden aus der Gruppe, die besteht aus N-Butylpyridinium-hexafluorophosphat, N-Ethylpyridinium-tetrafluoroborat, Tetrabutylphosphonium-tetrafluoroborat, 3-Butyl-1-methyl-imidazolium-hexafluoroantimonat, 3-Butyl-1-methyl-imidazolium-hexafluorophosphat, 3-Butyl-1-methyl-imidazolium-trifluoromethylsulfonat, Pyridinium-fluorosulfonat, Trimethylphenylammonium-hexafluorophosphat, 3-Butyl-1-methyl-imidazolium-tetrachloroaluminat, 3-Butyl-1-methyl-imidazoliumheptachloroaluminat, Trimethylphenylammonium-chlorid, 3-Ethyl-1-methylimidazolium-chlorid, Tetrabutylphosphonium-bromid, N-Butylpyridinium-chlorid, N-Ethylpyridinium-bromid, 3-Butyl-1-methyl-imidazolium-chlorid, Diethylpyrazolium-chlorid und Pyridinium-chlorhydrat.

7. Verfahren nach einem der vorhergehenden Ansprüche, in dem das Übergangsmetall ausgewählt wird aus der Gruppe, die besteht aus Ruthenium, Rhodium und Iridium.

8. Verfahren nach einem der vorhergehenden Ansprüche, in dem die Übergangsmetall-Verbindung ein Übergangsmetall-Komplex ist.

9. Verfahren nach einem der vorhergehenden Ansprüche, dadurch gekennzeichnet, daß das Metall an neutrale oder ionische Liganden gebunden ist.

10. Verfahren nach einem der vorhergehenden Ansprüche, in dem die Komplexe der Metalle der Gruppen 8 und 9 des PSE ausgewählt werden aus der Gruppe, die besteht aus RuH₄(PPh₃)₂, RhCl(PPh₃)₃, [Rh(Norbornadien)(PPh₃)₂]⁺[PF₆]⁻, IrCl((PPh₃)₃, HRh(CO)(PPh₃)₃, (C₅H₅)RhCl₂, IrCl(CO)(PPh₃)₂, [Rh(Norbornadien)(PPh₃)(Ph₂PCH₂COPh)]⁺[PF₆]⁻, [Rh(Norbornadien)(bisdiphenylphosphinoethan)]⁺[PF₆]⁻, [Rh(Norbornadien)(bisdiphenylarsinoethan)]⁺[PF₆]⁻, [Ru(Norbornadien)(C₅Me₅)]⁺[BF₄]⁻, [(C₆Me₆)RuCl₂]₂ und [(C₆H₆)RuCl₃Ru(C₆H₆)]⁺PF₆⁻.

11. Verfahren nach einem der vorhergehenden Ansprüche, in dem die Konzentration der Verbindung der Übergangsmetalle der Gruppen 8 bis 10 des PSE, bezogen auf das Ammonium- und/oder Phosphoniumsalz, 1 bis 500 mmol pro Liter beträgt.

12. Verfahren nach einem der vorhergehenden Ansprüche, in dem die katalytische Zusammensetzung ein organisches Lösungsmittel enthält.

13. Verfahren nach einem der vorhergehenden Ansprüche, in dem die Olefine 1-Buten, 2-Buten, 1-Penten, 2-Penten, 2-Methyl-2-buten, 2-Methyl-1-buten, 4-Methyl-2-pentene, 4-Methyl-1-penten, 2-Methyl-1-penten, 1-Hexen, 2-Hexene und 3-Hexene sind.

14. Verfahren nach einem der vorhergehenden Ansprüche, in dem die Kohlenwasserstoff-Phase von der polaren Phase abgetrennt wird, die mindestens einen Teil des Katalysators enthält, der mindestens zum Teil in den Isomerisierungs-Reaktor im Kreislauf zurückgeführt wird.

15. Verfahren nach einem der vorhergehenden Ansprüche, bei dem man zwischen -10 und 200°C unter einem Druck zwischen 0,1 MPa und 20 MPa arbeitet.

## Claims

1. A process for the isomerisation of double bond in olefins, in which said olefin is brought into contact with a catalytic composition comprising at least one compound of at least one transition metal from groups 8, 9 and 10 and at least one quaternary ammonium and/or phosphonium salt having general formula Q⁺A⁻ where Q⁺ represents a quaternary ammonium and/or quaternary phosphonium cation and A- represents an anion selected from the group formed by tetrafluoroborate, tetrachloroborate, hexafluorophosphate, hexafluoroantimonate, hexafluoroarsenate, dichlorocuprate, tetrachloroaluminate, trifluoromethylsulphonate, fluorosulphonate and trichlorozincate

2. A process according to any one of the preceding claims, in which the catalytic composition further comprises at least one anion selected from the group formed by heptachloroaluminate, decachloroaluminate, trichlorocuprate and tetrachlorocuprate.

3. A process according to any one of the preceding claims, in which the quaternay ammonium and/or phosphonium cation is selected from the group formed by cations with general formula
R¹R²R³R⁴N⁺
R¹ R²N=C R³ R⁴⁺
R¹ R² R³ R⁴P⁺
R¹ R²P=C R³ R⁴⁺ 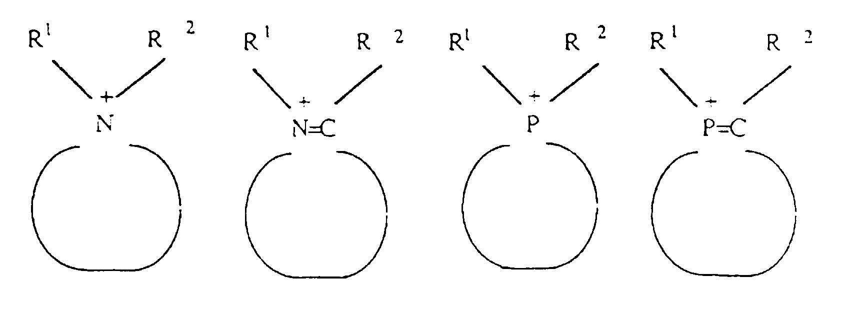
where R¹, R², R³ and R⁴, which may be identical or different, represent hydrogen, with the exception of NH₄+, and hydrocarbon residues containing 1 to 12 carbon atoms, and in which the cycles are constituted by 4 to 10 atoms.

4. A process according to any one of the preceding claims,in which the quaternay ammonium and/or phosphonium cation has one of the following general formula:
R¹R²N⁺=CR³-R⁵-R³C-N⁺R¹ R²
R¹ R²P⁺=CR³ -R⁵ -R³ =P⁺R¹ R²
where R¹, R² and R³ which may be identical or different, represent hydrogen or hydrocarbon residues containing 1 to 12 carbon atoms, and R⁵ represents an alkylene or phenylene residue.

5. A process according to any one of the preceding claims, in which the quaternary ammonium and/or phosphonium cation is selected from the group constituted by N-butylpyridinium, N-ethylpyridinium, 3-butyl-1-methylimidazolium, diethylpyrazolium, 3-ethyl-1-methylimidazolium, pyridinium, trimethylphenylammonium, 3-ethyl-1-methylimidazolium, and tetrabutylphosphonium.

6. A process according to any one of the preceding claims, in which the quaternary ammonium and/or phosphonium salt is selected from the group constituted by N-butylpyridinium hexafluorophosphate, N-ethylpyridinium tetrafluoroborate, tetrabutylphosphonium tetrafluoroborate, 3-butyl-1-methylimidazolium hexafluoroantimonate, 3-butyl--methylimidazolium hexafluorophosphate, 3-butyl-1-methylimidazolium trifluoromethylsulphonate, pyridinium fluorosulphonate, trimethylphenylammonium hexafluorophosphate, 3-butyl-1-methylimidazolium tetrachloroaluminate, 3-butyl-1-methylimidazolium heptachloroaluminate, trimethylphenylammonium chloride, 3-ethyl-1-methylimidazolium chloride, tetrabutylphosphonium bromide, N-butylpyridinium chloride, N-ethylpyridinium bromide, 3-butyl-1-methylimidazolium chloride, diethylpyrazolium chloride, and pyridinium hydrochloride.

7. A process according to any one of the preceding claims, in which the transition metal is selected from the group formed by ruthenium, rhodium and iridium.

8. A process according to any one of the preceding claims, in which the transition metal compound is a transition metal complex.

9. A process according to any one of the preceding claims, in which the metal is bonded to neutral or ionic ligands.

10. A process according to any one of the preceding claims, in which the complexes of metals from groups 8 or 9 are selected from the group formed by RuH₄(PPh₃)₂, RhCl(PPh₃)₃,[Rh(norbornadiene) (PPh₃)₂]⁺[PF₆]⁻, IrCl(PPh₃)₃, HRh(CO)(PPh₃)₃, (C₅H₅)RhCl₂, IrCl(CO)(PPh₃]₂, [Rh(norbornadiene) (PPh₃)(Ph₂PCH₂COPh)]⁺[PF₆]⁻, [Rh(norbornadiene) (bisdiphenyphosphinoethane)]⁺[PF₆]⁻, [Rh(norbornadiene) (bisdiphenylarsinoethane)]⁺[PF₆]⁻, [Ru(norbornadiene)(C₅Me₅]⁺[BF₄]⁻, [(C₆Me₆)RuCl₂]₂, [(C₆H₆)RuCl₃Ru(C₆H₆)]PF₆.

11. A process according to any one of the preceding claims, in which the concentration of the compound of transition metals from groups 8 to 10 is 1 to 500 mmoles per litre with respect to the ammonium and/or phosphonium salt.

12. A process according to any one of the preceding claims, in which the the catalytic composition comprises an organic solvent.

13. A process according to any one of the preceding claims, in which the olefins are 1-butene, 2-butene, 1-pentene, 2-pentene, 2-methyl-2-butene, 2-methyl-1-butene, 4-methyl-2-pentenes, 4-methyl-1-pentene, 2-methyl-1-pentene, 1-hexene, 2-hexenes and 3-hexenes.

14. A process according to any one of the preceding claims, in which the hydrocarbon phase is separated from the polar phase, said polar phase containing at least a portion of the catalyst being recycled at least in part to the isomerisation reactor.

15. A process according to any one of the preceding claims, carried out at -10°C to 200°C, at a pressure in the range 0.1 MPa to 20 MPa.
